# EUROPEAN PATENT SPECIFICATION

(11) **EP 2 408 407 B1**
(45) Date of publication and mention of the grant of the patent: **11.05.2016**
(21) Application number: 10710498.6
(22) Date of filing: 16.03.2010
(51) Int. Cl.: A61F 7/00, H05B 3/00, A61N 5/06, A61G 11/00, A61G 13/10, A61F 7/08, A61B 6/04

(54) **WARMING THERAPY DEVICE INCLUDING HEATED MATTRESS ASSEMBLY**
WÄRMENDE THERAPIEVORRICHTUNG MIT ERHITZTER MATRATZENANORDNUNG
DISPOSITIF DE THÉRAPIE DE RÉCHAUFFEMENT COMPRENANT UN ENSEMBLE MATELAS CHAUFFANT

(30) Priority: 18.03.2009 US 161184 P; 17.04.2009 US 170173 P
(43) Date of publication of application: 25.01.2012
(73) Proprietor: Draeger Medical Systems, Inc., Telford, PA 18969 (US)
(72) Inventor: PATEL, Kanti, King of Prussia PA 19406 (US); KHODAK, Andrei, Hatfield PA 19440 (US); SABOTA, Peter, D., Warminster PA 18974 (US); METT, Frank, Luebeck (DE); PHILLIPS, Mark, A., King of Prussia PA 19406 (US)
(74) Representative: McCartney, Jonathan William
(86) International application number: PCT/US2010/027379
(87) International publication number: WO 2010/107724

(56) References cited:
- EP-A2- 0 872 227
- GB-A- 836 776
- US-A- 4 825 868
- US-A- 5 138 138
- US-A- 5 759 149

## Description

### FIELD OF THE INVENTION

This present invention relates generally to an apparatus for performing warming therapy on medical patients. More particularly, the present, invention relates to an apparatus for heating a mattress in a warming therapy device, wherein the heating apparatus is X-ray transparent.

### BACKGROUND OF THE INVENTION

Conventional warming therapy devices achieve patient warming through the use of air pumps and convective and radiant heaters. Some existing devices also use a conductive method of warming therapy, or heaved matress. One such device (which is manufactured by the present assignee and sold under the name Babytherm®) utilizes an electric resistive heater attached to a metal plate which supports a thermally conductive gel mattress. The metal plate design provides some degree of uniform temperature distribution across the surface of the mattress, however, it is also non-transparent to X-rays. So, an X-ray cassette can only be placed over the mattress support containing the metal plate, which may interfere with the care of the patient disposed on the mattress. Other conventional warming devices including conductive warming utilize X-ray transparent carbon fiber material instead of metal plate.

US Patent No. 5,138,138 discloses a heating system for an operating table comprising a multi-layer heating pad. The heating pad comprises four heating segments, arranged end-to-end in a single row, that are heated and temperature controlled through a control and regulating unit.

For example, U.S. Patent Nos. 6,814,889 and 6,974,935 discuss a heated mattress device 1 which includes a conductive layer 3 with carbon particles. Carbon fiber is electrically conductive, and thus allows a similar distribution of heat as provided by the metal plate, and also permits the placement of an X-ray cassette below the carbon fiber layer. However, such structures often leaves artifacts on the X-ray image due to non-X-ray transparent temperature control sensors, buses and connecting wires disposed in the area around the carbon fiber layer.

The present invention overcomes the deficiencies of conventional warming therapy devices through the use of a heated mattress assembly as claimed in claim 1.

### BRIEF DESCRIPTION OF THE DRAWINGS

Figure 1 is perspective view of a warming therapy device according to the present invention.
Figure 2 is an overhead perspective view of the warming therapy device of Figure 1.
Figure 3 is a simplified side cross-section view of the warming therapy device of Figure 1.
Figure 4 is a top view with detail of a heating element according to first exemplary embodiment of the present invention.
Figure 5 is a simplified side cross-section view of a warming therapy device according to an exemple.
Figure 6 is a top view of a heating element according to an exemple.
Figure 7 is a side view of the heating clement of Figure 6.
Figure 8 is a detail side view of the heating element of Figure 6, shown along Detail A in Figure 7.
Figure 9 is a top cress-section view of the heating element of Figure 6 which shows the thermocouple grid and an associated controller.
Figure 10 is a top cross-section view of a heating element according to an exemplary embodiment of the present invention.
Figures 11 is a top cross-section view of a heating element according to an exemplary embodiment of the present invention.
Figure 12 is top cross-section view of a heating element according to an example.
Figure 13 is a Mock diagram of an oxygen control system according to to an example.

### DETAILED DESCRIPTION

The present invention relates to a warming therapy device (e.g., incubator, warmer, etc.) including a heated mattress tray assembly. In particular, the warming therapy device includes a resistive or radiolucent heater disposed within a mattress tray assembly for providing heating of a patient disposed on the mattress tray assembly. In an example the warming therapy device includes a segmented carbon fiber heater coupled to an oxygen control device and disposed within a mattress tray assembly for providing heating of a patient situated on the mattress tray assembly.

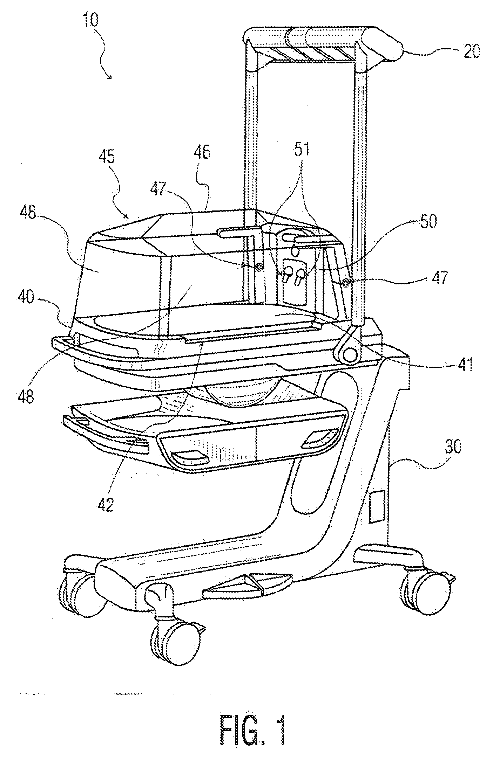

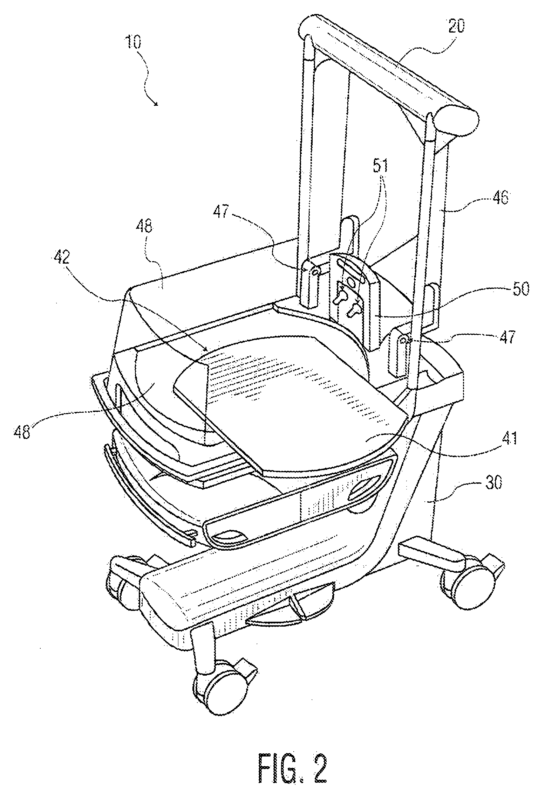

Figures 1 and 2 show a warming, therapy device 10 according to the present invention. The warming therapy device 10 includes a radiant heater head 20, and a patient support assembly 30 including a mattress tray assembly 40. The mattress tray assembly 40 may include a hood 45 which has a top portion 46 which pivots about one or more axes 47. The hood 45 may also include one or more sidewalls 48 which may be slideable, removable, pivotable or rotatable. The mattress tray assembly 40 includes a mattress tray 41 disposed within a recessed support base 42.

The mattress tray 41 may be made rotatable within the support base 42 up to three hundred and sixty degrees (360°). Figure 2 specifically show the mattress tray 41 rotated approximately ninety degrees (90°) with respect to the position shown in Figure 1. Figure 2 also shows the top portion 46 of the hood 45 rotated up so that it is approximately ninety degrees (90°) with respect to the mattress tray 41. In the exemplary embodiment shown in Figure 2, the side walls 48 of the hood 45 are capable of sliding vertically within a portion of the mattress tray assembly 40, so that they may become disposed, partially or completely, below the plane of the mattress stray 41.

The warming therapy device 10 may optionally include backplane 50, to which ventilation hoses and other devices may be coupled through, for example, interconnection nozzles 51. The backplane 50 may also include one or more oxygen sensors disposed therein for sensing an oxygen level inside the hood 45, as explained in detail below.

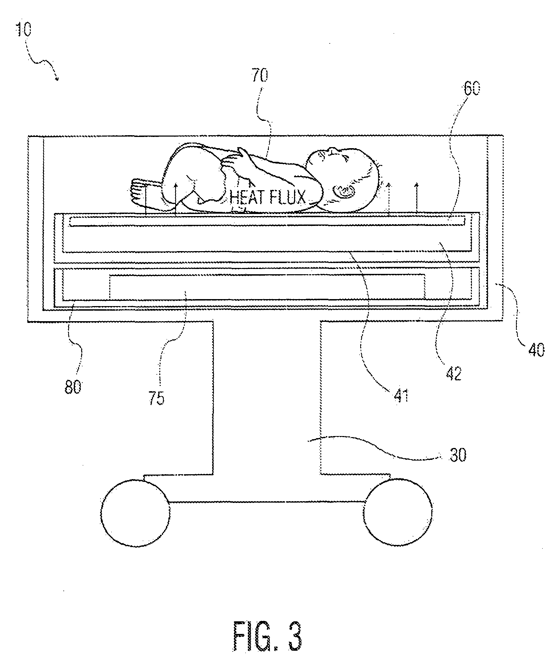

Figure 3 shows a simplified side cross-sectional view of the warming therapy device 10 shown in Figures 1 and 2. The mattress tray assembly 40, including mattress tray 41 and mattress 42, are shown disposed on the patient support assembly 30. Disposed within the mattress 42 is an electric resistive heating element 60 for providing heat to a patient 70 disposed on the mattress. As discussed in detain below, the heating element 60 may be formed of a single piece, on may be divided into a plurality of separate portions, or segments, A utility tray 80 may be disposed beneath the mattress tray 41, and may house an X-ray cassette 75. The X-ray cassette 75 may be used, for example, in performing X-ray examinations of the patient 70, as is well known in the art.

The resistive heating element 60 may be formed or a substantially flat member or plurality of substantially flat members (segments) which extend through some or all of the mattress 42. For example, if the mattress 42 is substantially rectangular in shape as shown in Figures 1 and 2, the resistive heating element 60 may be formed as a rectangle with similar dimension, or a plurality of rectangular segments. The resistive heating element 60 may be formed of materials with similar structure parameters as the maternal for the mattress 42, such as: (1) thin metal cloth or foil (e.g., Aluminum foil), (2) non-metallic conductive film (e.g., graphite or graphene film), or (3) cloth made from non-metallic conductive yarn (e,g., carbon fiber or carbon nanotube material), insuring the overall softness and flexibility of the mattress are maintained. Carbon nanotube material is available from various commercial sources, including from CNT Technologies (Seattle, WA) and Nanocomp Technologies (Concord, NH). The resistive heating element 60 may be placed within the mattress 42 so as to efficiently transmit heat to the patient 70, but also so as to prevent excessive heating of the members below the mattress. For example, the resistive heating element 60 may be placed in the mattress 42 at a position closer to the patient 70, so that the material of the mattress acts as an insulator with respect to the X-ray cassette 75, and any other devices located beneath the mattress 42.

As mentioned above, non-metallic conductive materials may be used for the resistive heating element 60. For example, materials using carbon nanotubes may be utilized to achieve an increase in electrical and thermal conductivity. Materials such as graphene may also be used for the resistive heating element 60. Grapheme in particular shows electrical conductivity even as one (1) atom layer thick film, and is virtually undetectable by microscope. Conducive plastics, such as optically transparent vacuum metalized polyethylene, may also be used for the resistive heating element 60. However, in order to insure effective X-ray imaging of the patient 70, any material used for the resistive heating element 60 should be X-rsy transparent, which is the case for some conductive plastics and thin metal cloth. The X-ray cassette 75 is an optional element and in cases where X-ray capacity is not needed the resistive heating element 60 may be formed of materials which are not necessarily X-ray transparent.

The thermal capacity of the mattress 42 may be made sufficiently large so as to allow continued warming of the patient 70 even after the electric heating element 60 is turned off. Certain gels, such as polyarethane and silicone gels, may be used for the forming the entirely, or potion of, the mattress 142, as they have a high thermal capacity. The previously-mentioned Babytherm® product manufactured by the present assignee includes such a gel within the mattress. However, those of ordinary skill in the art will realize that other materials (e.g., thermally conductive plastic foam) are also suitable for forming the mattress 42, or some portion thereof.

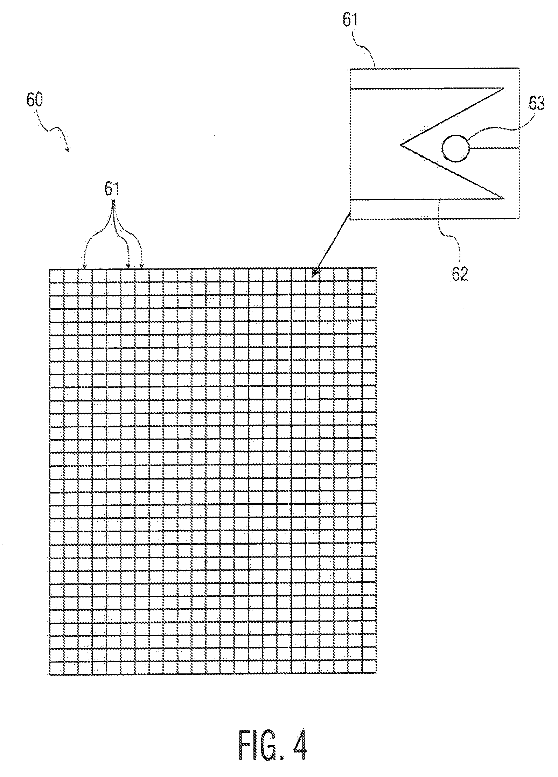

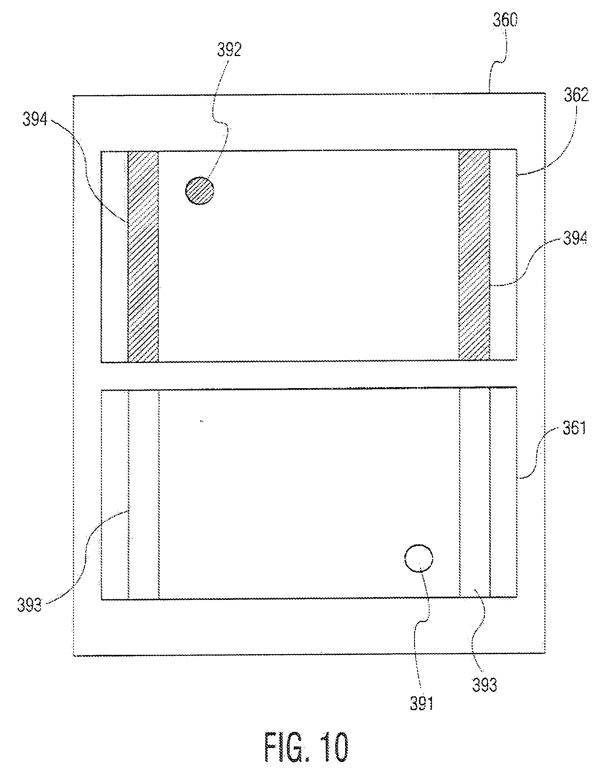

Figure 4 shows details of a heating element 60 according to the present intention. As shown, the resistive heating element 60, or each segment thereof (when using a segmented heating clement such as shown and described in connection with Figures 10-12 below), is formed as a matrix structure, so as to provide for substantially uniform temperature distribution across the heating element or each separate segment. Such a structure provides substantially uniform temperature distribution across the surface of the heating element. In particular, the resistive heating element 60 is divided into a plurality of cells 61, each containing a heating element 62 controlled by a sensor 63. This type of configuration allows each cell 61 to be powered on or off depending upon the temperature measured by the sensor 63 associated with that cell. As noted above, X-ray transparent materials, such as conductive plastics and thin metal cloth, may be selected for forming the heating elements 62 and sensors 63 in order to permits effective X-ray imaging of the patient 70. As an alternative to the cells 61 including heating elements 62 and sensors 63 discussed above, Positive Temperature Coefficient (PTC) heaters may be used in a Matrix arrangement for the resistive heating clement 60. PTC heaters are typically formed from a especial compound which may increase the thermal resistance of the heater at a particular temperature lever This characteristic provides a sell-controlling mechanism whereby the heater temperature can be stabilized at that particular level. Additionally, materials with a strong dependence of electrical conductivity on temperature (such as carbon nanotube materials) can be used in a matrix structure simultaneously, as heaters and temperature sensors.

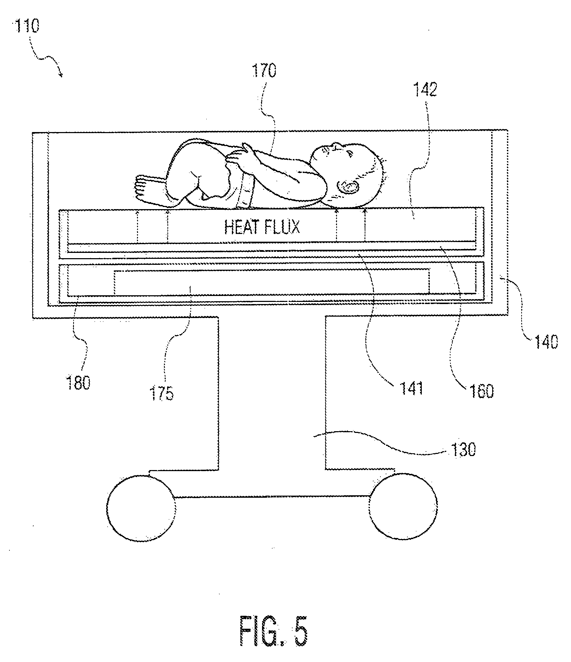

Figure 5 shows a simplified side cross-sectional view of a warming therapy device 110 according to to an example. The warming therapy device 110 is similar to the warming therapy devices 10 discussed above, and like reference numerals denote like elements. The warming therapy device 110 may include as resistive heating element 160, which is similar in structure to the resistive heating element 60 shown in Figure 4.

The warming therapy device 110 includes a mattress tray assembly 140, including a mattress tray 141 and mattress 142, which are disposed on a patient support assembly 130. As opposed to the waring therapy device 10, an electric resistive heating element 160 is disposed on the mattress tray 141, for providing heat to a patient 170 disposed on the mattress 142. A utility tray 180 may be disposed beneath the mattress tray 140, and may house an X-ray cassette 175. The X-ray cassette 175 may be used, for example, in performing X-ray examinations of the patient 170, as is well known in the art.

The resistive, heating element 160 may be a substantially flat member which extends through some or all of the mattress tray 141. For example, if the mattress tray 141 is substantially rectangular in shape as shown in Figures 1 and 2, the resistive heating element 160 may be formed as a rectangle with similar dimensions. As noted above, the resistive heating element 160 may be formed of materials such as: (1) thin metal cloth or foil (e.g., Aluminum foil), (2) non-metallic conductive film (e.g., graphite or graphene film), or (3) cloth made from non-metallic conductive yarn (e.g., carbon fiber or carbon nanotube material). In the second exemplary embodiment, the material for the mattress 142 may be selected to have high thermal to as to effectively transmit heat from the resistive heating element top the patient 170. For example, the mattress 142 may be formed of partially or completely of thermally conductive gel or plastic foam.

As with the resistive heating element 60 discussed above, non-metallic conductive materials may be used for the resistive heating element 160. For example, materials using carbon nanotubes may be utilized to achieve an increase if electrical and thermal conductivity. Materials such as graphene may also be used for the resistive heating element 160. Graphene in particular shows electrical conductivity even as one (1) atom layer thick film, and is virtually undetectable by microscope. Conductive plastics, such as optically transparent vacuum metallized polyethylene, may also be used for the resistive heating element 160. However, in order to insure effective X-ray imaging of the patient 170, any material used for the resistive heating element 160 should be X-ray transparent, which is the case for some conductive plastics and this metal cloth. Those of ordinary skill will realize that the X-ray cassette 175 is not a necessary element of the above-described exemplary embodiment, and in cases where X-ray capacity is not needed the resistive heating element 160 may be formed of materials which are not necessarily X-ray transparent.

The thermal capacity of the mattress 142 may be made sufficiently large so as to allow continued warming of the patient 170 even after the electric heating element 160 is turned off. Certain gels, such as polyurethane and silicone gels, may be used for the forming the entirely, or a portion of, the mattress 142, as they have a high thermal capacity. The previously-mentioned Babytherm® product manufactured by the present assignee includes such a gel within the mattress. However, those of ordinary skin in the art will realize that other materials (e.g., thermally conductive plastics foam) are also suitable for forming the mattress 142, or some portion thereof.

Again, as with the resistive heating element 60 discussed above, the resistive heating element 160 may be formed as a matrix structure, as shown in Figure 4. Such a structure provides substantially uniform temperature distribution across the surface of the heating element 160. As also noted above, PTC heaters may be used in a matrix arrangement for the resistive heating element 160.

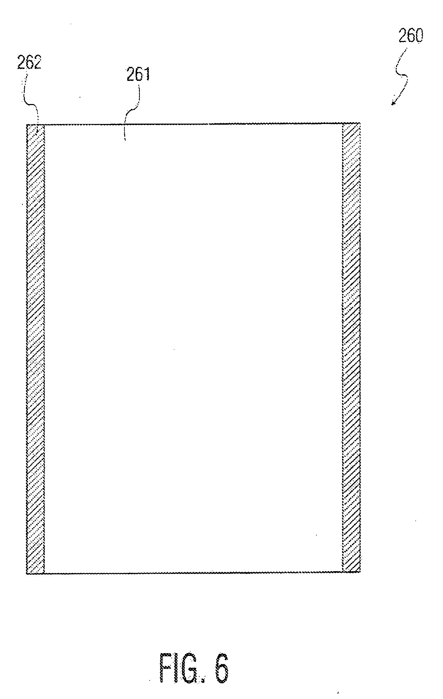

Figure 6 shows a top view of a heating element 260 according to an example. This heating element 260 may be used in connection with a mattress tray assembly (including a mattress tray and mattress) and warming therapy device, such as the mattress tray assemblies 40 and 140, and warming therapy devices 10, 110. In particular, the heating element 260 may be placed in close proximity to a patient (such as in a mattress or mattress tray assembly), in order to provide heating of the patient.

When, for example, a patient is disposed on or in close proximity to the heating element 260, the temperature directly below the patient (at the interface) increases due to the fact that heat dissipation to the ambient environment is at least partially blocked by the patient's body. The heating element 260 overcomes this increased heating problem through the use of a thermocouple grid (i.e., temperature sensing array), as explained below.

As shown in an example Figure 6, the heating element 260 may comprise a radiolucent heating element 261, such as a carbon film heater (made from, for example, carbon-filled polymer film or carbon nanotube fllm), which is bordered on at least two (2) sides by electrically conductive bus bars 262. The bus bars 262 may be coupled to a source of electrical power, such as a voltage source. The bus bars 262 extend through the entire cross-section of the heating element 261, whereas the radiolucent heating element may be comprised of various layers, as discussed below. The bus bars 262 may be formed of an electrically conductive non-radiolucent materials (such as, for example, Copper mesh. Copper plated film), or radiolucent materials (e.g., carbon fiber), of a blend of both.

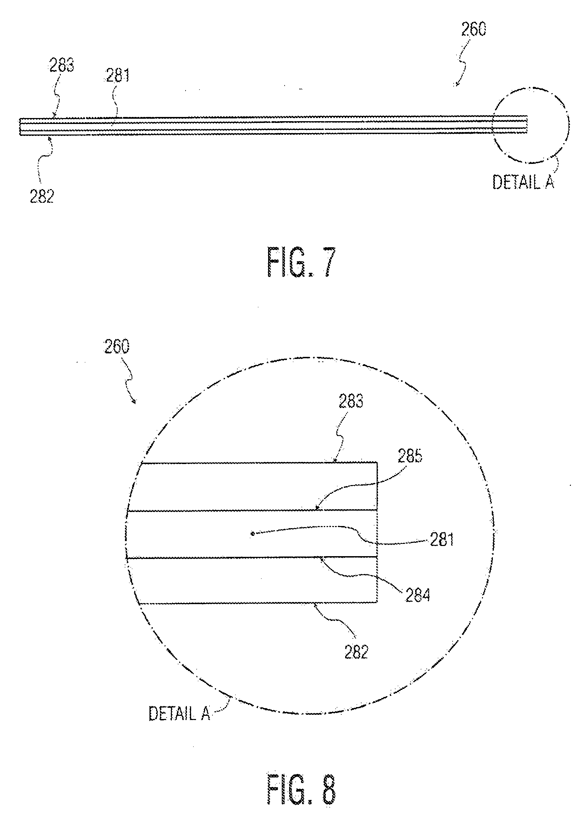

Figure 7 shows a side view of the heating element 260. As shown, the heating element 260 may be comprised of at least three (3) layers, an insulating layer 281 which is sandwiched by two (2) support layers 282, 283. The insulating layer 281 may be formed of thin, flexible electrically insulating plastic (e.g., polyester, polyurethane) film. The support layer 282 may be formed of foam materials such as polyurethane or polyester foam. The support layer 283 may be formed of a material for improving patient comfort, such as an expanded polyester pad. The thickness of the support layers 282, 283 contributes to the heat conducted to the patient. Ideally, the support layer 282 offers an insulating property to prevent heat loss from the bottom surface of the heating element 284, whereas support layer 283 is more thermally conductive than support layer 282, thus providing patient comfort and more efficient heat transfer to the patient. All the layers of the heating element 260 may be sealed with a waterproof outer covering that may be made out of synthetic or natural fabric materials. In such an example, the patient would lie on top of the waterproof outer covering.

Figure 8 shows a detail side view of the heating element 260, along Detail A shown in Figure 7. Figure 8 shows the insulating layer 281, and support layers 282, 283, as well as a carbon film heater layer 284 (sandwiched between the support layer 282 and the insulating layer 281) and a thermocouple grid layer 285 (sandwiched between the support loyer 283 and the insulating layer 281). Thus, the heating element 260 may be formed of at least five (5) layers: a first support layer 282, carbon film heater layer 284, an insulating layer 281, a thermocouple grid layer 285, and a second support layer 283. Both the carbon film heater layer 284 and the thermocouple grid layer 285 may be formed of radiolucent carbon film. One of the benefits of using radiolucent carbon film for the heater layer 284 and the thermocouple grid layer 285 is that it is X-ray transparent, thereby allowing X-ray procedures to be performed on a patient disposed on or near the heating element 260.

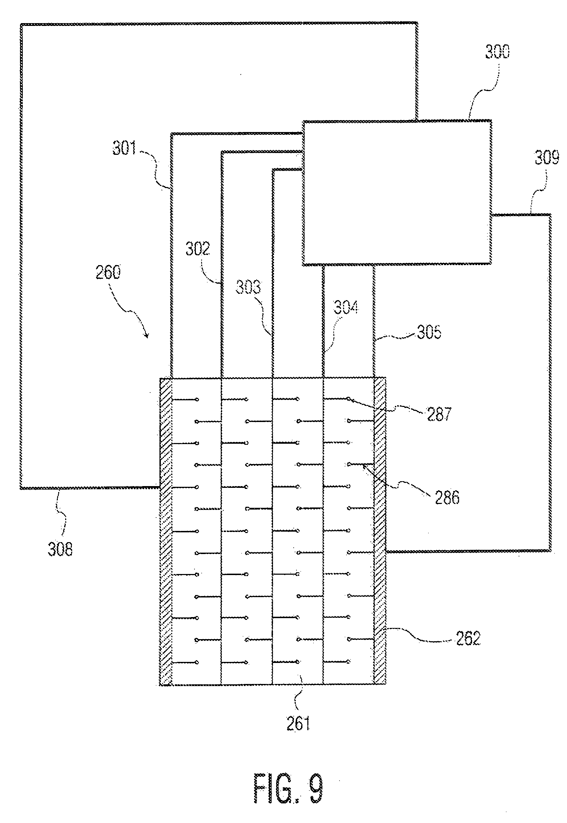

Figure 9 shows a top cross-sectional view of the heating element 260 which shows the structure of the thermocouple grid layer 285. The thermocouple grid layer 285 may be comprised of a series of electrically conductive leads 286 which terminate in respective thermocouples 287. The thermocouples 287 operate to sense the temperature at various portions of the heating element 260, and may be used to maintain a pre-selected temperature for the heating element. The spacing of the thermocouples 287 along the thermocouple grid layer is preferably less than the contact area of the patient (disposed on the mattress 242) with the heating element 260, so that at least one thermocouple is always covered by the patient. Such an arrangement also permits the temperature at the interface between the heating element 260 and the patient to be maintained at the pre-selected value. The thermocouples 287 may be made from radiolucent carbon nanotube materials. Each thermocouple 287 provides temperature information to a mattress controller 300. The mattress controller 300 regulates the electric power supplied to the heating element 260 via power bus bars 262, based on the temperature information provided by the thermocouples 287 and a predetermined mattress temperature (which may be set by a user or caregiver). The mattress controller compares the predetermined temperature against the data from the thermocouples 287 and controls the power to the heating element 260. The temperature at each bus of thermocouples 287 may be measured on one or more control lines 301-305, and provided to the controller 300 for comparison to the predetermined temperature. Power : control lines 308, 309 are coupled to the controller 300 and the power bus bars for providing regulation of the power supplied to the bus bars, and thus the heat of the heating element 260.

Although the mattress tray assemblies 40, 140 are shown and described above with reference to an associated warming therapy devices 10, 110 of specific configurations, those of ordinary skill in the art will realize that the mattress tray assemblies 40, 140 may be integrated into any suitable incubator, warmer, medical treatment device or other equivalent apparatus.

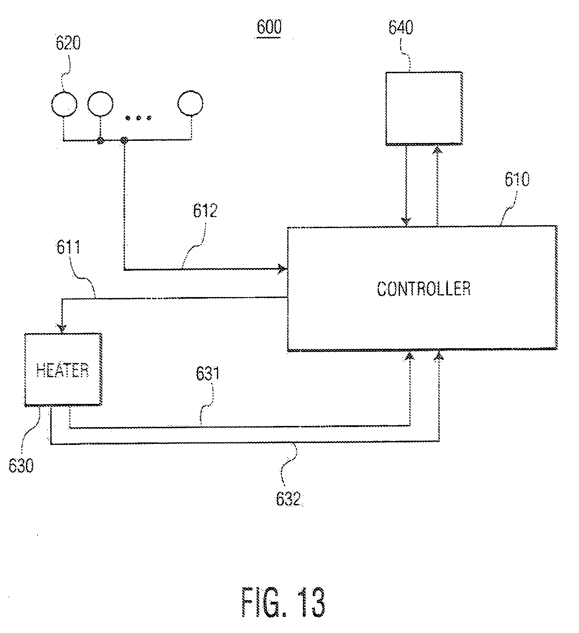

Figure 10 shows a heating element 360 according to an examplary embodiment of the present invention. The heating element 360 may be used in connection with a mattress tray assembly (including a mattress tray and/or mattress) and/or a warming therapy device, such as the mattress tray assemblies 40, 140, and warming therapy devices 10, 100, discussed above. The heating element 360 may be divided into a plurality of segments in the third exemplary embodiment, including a first segment 361 and a second segment 362. The first segment 361 may be coupled to a first sensor 391 for sensing the temperature of the first segment, and providing a control signal for use in regulating such temperature to a system controller 610 (as shown in Fig. 13). Similarly, the second segment 362 may be coupled to a second sensor 392 for sensing the temperature of the second segment, and providing a control signal to the system controller 610 for use in regulating such temperature. The first and second segments 361, 362 may also include respective power rails or buses 393, 394 for receiving power from an external voltage or current source, which may be part of the system controller 610. As noted above, the first and second segments 361, 362 are preferably radiolucent (e.g., X-ray transparent or translucent) and designed using primarily carbon fiber materials (e.g., carbon films, carbon nanotubes, etc.).

Each of the first and second segments 361, 362 of the heating element 360 may be power-limited according specific standards. For example, each of the segments 361, 362 may have an upper power limit of 15VA, thereby permitting a total power of the heating element 360 to be up to 30VA. As noted above, current IEC guidelines place an upper limit of 15 VA per circuit, and thus the heating element 360 meets IEC guidelines.

The heating element 360 may also include an Oxygen Control Device (OCD) which controls the power supplied to the heating segments 361, 362 between lower and upper limits. For example, a lower power limit may be the IEC guideline for oxygen rich environments of 15VA, and the upper limit may be greater than 50VA (for environments which are not oxygen rich). The upper limit may be set so as to insure that the mattress surface temperature does not exceed 42°C. The OCD may additionally control the amount of oxygen supplied to the patient 70 disposed on the mattress 42 (when utilizing the heating element 360 in connection with the warming therapy device 10).

in operation, a user (e.g., health care professional) selects a desired mattress temperature (e.g., 39° C), and the OCD controls the power supplied to the heating segments 361, 362, and the oxygen supply, so that the mattress (e.g., mattress 42) reaches the desired temperature in the shortest possible time given conditions. For example, if oxygen is not immediately required for the patient during warm up, the OCD would allow maximum power to be supplied to the heating segments 361, 362 (e.g., 70-100VA) until the desired temperature was reached. Once the desired temperature was reached, the OCD would initiate supply of oxygen to the patient, and would maintain the temperature at the desired level through switching the power to the heating segments 361, 362 on and off at power levels at or below IEC limits (e.g., 15 VA or less). However, if oxygen is required for the patient during warm up, the OCD would regulate the power supplied to the heating segments 361, 362, so as not to exceed 15VA per segment, which would necessarily increase the warm up time.

Figure 13 shows a control system 600 for use in connection with the heating element 360 and warming therapy devices 10, 110. The control system 600 includes a system controller 610, one or more oxygen sensors 620, a heating element 630 (erg., heating element 360 described above), and a display and control unit 640. The system controller 610, which may comprise a specially-programmed Central Processing Unit (CPU) or the like, is adapted to supply power to the heating element 630 over first control line 611, and receive one or more control signals from the one or more oxygen sensors 620 over second control line 612. The system controller 610 is also adapted to receive one or more control signals on first and second sensor control lines 631, 632 from the heating element 630. The system controller 610 may also communicate with the display and control unit 640, to display information thereon, and to receive information wherefrom. For example, the display and control unit 640 may comprise a touch screen display which is capable of accepting user commands and displaying information,

The first control line 611 may be coupled to the power rails or buses of the heating element 630 (e.g., buses 393, 394 of the heating element 360), to enable the controller 610 to supply power to the heating element. Similarly, the second control line 612 may be coupled to the backplane of a warming therapy device (e.g., backplane 51 of the warming therapy device 10), which may include one or more oxygen sensors (e.g., oxygen sensors 630) as noted above. The first sensor control line 631 may be coupled to a first sensor of the heating element 630 (e.g., sensor 391), and the second sensor control line 632 may be coupled to a second sensor of the heating element (e.g., sensor 392). The system controller 610 may be used to set a predetermined mattress temperature for monitoring purposes. Such a predetermined temperature may be set (and changed) by a health care professional using, for example, the display and control unit 640.

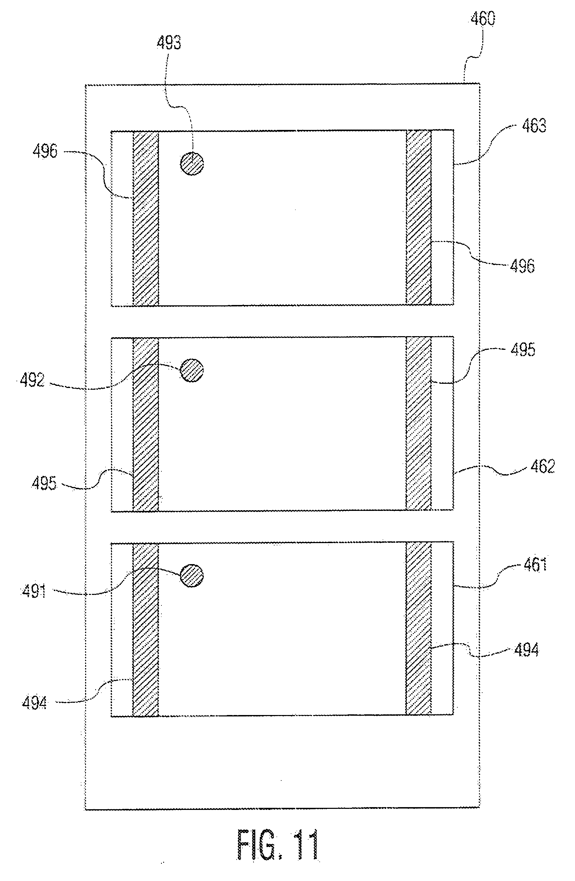

Figure 11 shows a simplified top cross-section view of a heating element 460 according to an exemplary embodiment of the present invention. The heating element 460 is similar to the heating element 360, according to the third exemplary embodiment in several respects, and in most cases, like reference numerals denote like elements. The heating element 460 may be used in connection with a mattress tray assembly (including a mattress tray and/or mattress) and/or a warming therapy device, such as the mattress tray assemblies 40, 140, and warming therapy devices 10, 100, discussed above.

The heating element 460 is divided into a plurality of segments, including a first segment 461, a second segment 462, and a third segment 463. The first segment 461 may be coupled to a first sensor 491, the second segment 462 may be coupled to a second sensor 492, and the third segment 463 may be coupled to a third sensor 493 ail for sensing the respective temperatures of the first through third segments, and providing control signals for use in regulating such temperatures to a system controller (e.g., system controller 610 shown in Fig. 13). The first through third segments 461, 462, 463 may also include respective powder rails or buses 494, 495, 496 for receiving power from an external voltage or current source, which may be part of the system controller (e.g., system controller 610). As noted above, the first through third segments 461, 462, 463 are preferably radiolucent (e.g., X-ray transparent or translucent) and designed using primarily carbon fiber materials (e.g., carbon films, carbon nanotubes, etc.).

The heating element 460 further reduces the appearance of artifacts on an X-ray by routing the power rails 494, 495, 496 outside the X-ray area (i.e., the central portion of the heating element 460). Additionally, the heating element 460 provides three (3) times the heating power of a single segment heater, due to the presence of three independently controllable heating segments. Those of ordinary skill in the art will realize that this scalability concept may be expanded to increase heating power by increasing the number of segments in the heater. For example, a heater witch four (4), five (5) or six (6) segments may be used to increase heating power.

The heating element 460 operates similarly to the heating element 360 in that a user (e.g., health care processional) selects a desired mattress temperature (e.g., 39°C), and the OCD controls the power supplied to the heating segments 461, 462, 463 and the oxygen supply, so that a mattress reaches a desired temperature in the shortest possible time given conditions. The main difference being that the OCD limits the power supplied to each of the heating elements 461,462,463 to 15 VA or less when oxygen is being supplied. However, the presence of three separate heating segments 461, 462, 463 permits a maximum power limit of 45 VA in oxygen rich environments (as opposed to 30 VA in the heating element 360).

The heating element 460 may be controlled by a control system 600 such as is shown in Figure 13. One difference would be the addition of a third sensor control line for coupling between one of the sensors 491, 492, 493 and the system controller 610. With the heating element 460, the first control line 611 may be coupled to the power rails or buses 494, 495, 496 to enable the controller 610 to supply power to the heating element 630. Similarly, the second control line 612 may be coupled to the backplane of a warming therapy device (e.g., backplane 51 of the warming therapy device 10), which may include one or more oxygen sensors (e.g., oxygen sensors 630) as noted above. The first sensor control line 631 may be coupled to the first sensor 491 of the heating element, the second sensor control line 632 may be coupled to the second sensor 492, and a third sensor control line (not shown) may be coupled to the third sensor 493. The system controller 610 may be used to set a predetermined mattress temperature for monitoring purposes. Such a predetermined temperature may be set (and changed) by a health care professional using, for example, the display and control unit 640.

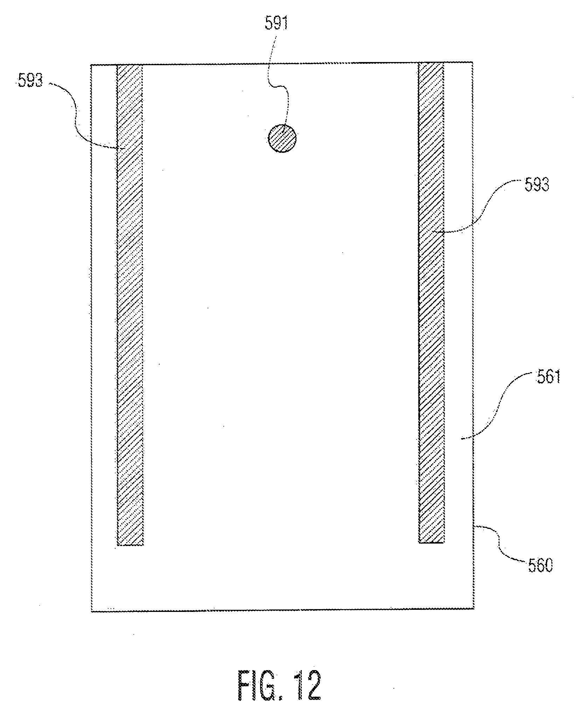

Figure 12 shows a simplified top cross-section view of a heating element 560 according to an example. The heating element 560 is simitar to the heating elements 360 and 460 in several respects, and in most cases, like reference numerals denote like elements. The heating element 560 may be used in connection with a mattress tray assembly (including a mattress tray and/or mattress) and/or warming therapy device, such as the mattress tray assemblies 40.140, and warming therapy devices 10, 100, discussed above.

Alternatively, from the heating elements 360 and 460, the heating element 560 is formed of a single segment 561. However, like the heating elements 360 and 460, the heating element 560 includes a first sensor 591 for sensing the temperature of the segment, and providing a control signal for use in regulating such temperature to a system controller (e.g., system controller 610 shown in Fig. 13). The heating element 560 may also include power rails or buses 593 for receiving power from an external voltage or current source, which may be part of the system controller (e.g., system controller 610). As noted above,the single segment 561 is preferably radiolucent (e.g., X-ray transparent or translucent) and designed using primarily carbon fiber materials (e.g., carbon films, carbon nanotubes, etc.).

The heating element 560 operates similarly to the heating elements 360 and 460 described above, in that a user (e.g., health care professional) selects a desired mattress temperature (e.g., 39°C), and the OCD controls the power supplied to the heating segment 561, and the oxygen supply, so that a mattress reaches a desired temperature in the shortest possible time given conditions. The main difference being that the OCD limits the power supplied to the single heating element 561 to 15 VA or less when oxygen is being supplied. Thus, the heating element 560 permits a maximum power limit of 15 VA in oxygen rich environments (as opposed to 30 VA in the heating element 360 and 45 VA in the heating element 460).

The heating element 560 may be controlled by a control system 600 such as is shown in Figure 13. One difference would be that only one of the first and second sensor control lines 631, 632 would be used to connect the system controller 610 to the heating element 630, due to the fact that the heating element includes only one sensor 591. With the heating element 560, the first control line 611 may be coupled to the power rails or buses 593 to enable the controller 610 to supply power to the heating element 630. Similarly, the second control line 612 may be coupled to the back plane of a warming therapy device (e.g., backplane 51 of the warming therapy device 1O), which may include one or more oxygen sensors (e.g., oxygen sensors 630) as noted above. The first sensor control line 631 may be coupled to the sensor 591 of the heating element 560. The system controller 610 may be used to set a predetermined mattress temperature for monitoring purposes. Such a predetermined temperature may be set (and changed) by a health care professional using, for example, the display and control unit 640.

Although sensors 391, 392, sensors 491, 492, 493 and sensor 591 are shown in specific locations on the respective heating elements 360. 460. 560, those of ordinary skill will realize that such sensors may be disposed at any suitable location on or near the heaters, so long as accurate temperature readings can be obtained thereby. Further, although the heating elements 360_{,} 460, 560 described above show heating segments of one, two and three portions, those of ordinary skill will realize that the present invention is not so limited, and that: heaters with four or more segments are contemplated by the present invention. Finally, although the heating elements 360, 460, 560 are shown and described above with reference to associated warming therapy devices 10, 110 of specific configurations, those of ordinary skill in the art will realize that the heating elements 360, 460, 560 may be integrated into any suitable mattress, incubator, warmer, medical treatment device or other equivalent apparatus.

Although the invention has been described in terms of exemplary embodiments, it is not limited thereto. Rather, the appended claims should be construed broadly to include other variants and embodiments of the invention which may be made by those skilled in the art without departing from the scope and range of equivalents of the invention. This disclosure is intended to cover any adaptations or variations of the embodiments discussed herein.

## Claims

1. An apparatus comprising:
a patient support assembly;
a mattress (42) disposed on the patient support assembly; and
at least one heating element (60) formed of a plurality of heating cells (61) disposed within the mattress,
each of the plurality of heating cells (61) comprising at least one substantially flat heating element (62) which may be individually powered on or off, and at least one temperature sensor (63),
**characterized in that**:
said at least one heating element (60) is divided into a plurality of heating cells (61) so as to form a substantially flat matrix.

2. The apparatus of claim 1, wherein the plurality of heating cells are arranged adjacent to one another in a matrix.

3. The apparatus of claim 1, further comprising:
a mattress tray for receiving the mattress, said mattress tray being coupled to the patient support assembly.

4. The apparatus of claim 3, further comprising an X-ray cassette disposed beneath the mattress tray.

5. The apparatus of claim 1, wherein each of the plurality of heating cells are formed from X-ray transparent material.

6. The apparatus of claim 1, wherein the at least one heating element is formed of a plurality of heating segments.

7. The apparatus of claim 6, wherein the at least one heating element is formed of at least two separate heating segments.

8. The apparatus of claim 6, wherein the at least one heating element is formed of at least three separate heating segments.

9. The apparatus of claim 1, further comprising:
a mattress tray assembly (40) coupled to the patient support assembly, said mattress tray assembly including a mattress tray (41) for holding a mattress (42), wherein the plurality of heating cells (61) are disposed within the mattress.

10. The apparatus of claim 9, wherein the at least one heating element is formed of at least two separate heating segments.

11. The apparatus of claim 9, wherein the at least one heating element is formed of at least three separate heating segments.

## Patentansprüche

1. Vorrichtung, umfassend:
eine Patiententrägeranordnung;
eine Matratze (42), die auf der Patiententrägeranordnung angeordnet ist; und
mindestens ein Heizelement (60), das aus einer Mehrzahl von Heizzellen (61) gebildet wird, welches in der Matratze angeordnet ist,
wobei jede von der Mehrzahl der Heizzellen (61) mindestens ein im Wesentlichen flaches Heizelement (62) aufweist, das individuell an- und ausgeschaltet werden kann, und mindestens einen Temperatursensor (63),
**dadurch gekennzeichnet, dass**:
das mindestens eine Heizelement (60) in eine Mehrzahl von Heizzellen (61) unterteilt ist, um so eine im Wesentlichen flache Matrix zu bilden.

2. Vorrichtung nach Anspruch 1, wobei die Mehrzahl der Heizzellen aneinander angrenzend in einer Matrix angeordnet sind.

3. Vorrichtung nach Anspruch 1, ferner umfassend:
eine Matratzenschale zum Aufnehmen der Matratze, wobei die Matratzenschale mit der Patiententrägeranordnung verbunden ist.

4. Vorrichtung nach Anspruch 3, ferner aufweisend eine Röntgenkassette, die unter der Matratzenschale angeordnet ist.

5. Vorrichtung nach Anspruch 1, wobei jede von der Mehrzahl der Heizzellen aus einem für Röntgenstrahlen durchlässigen Material gebildet ist.

6. Vorrichtung nach Anspruch 1, wobei das mindestens eine Heizelement aus einer Mehrzahl von Heizsegmenten gebildet ist.

7. Vorrichtung nach Anspruch 6, wobei das mindestens eine Heizelement aus mindestens zwei getrennten Heizsegmenten gebildet ist.

8. Vorrichtung nach Anspruch 6, wobei das mindestens eine Heizelement aus mindestens drei getrennten Heizsegmenten gebildet ist.

9. Vorrichtung nach Anspruch 1, ferner umfassend:
eine Matratzenschalenanordnung (40), die mit der Patiententrägeranordnung verbunden ist, wobei die Matratzenschalenanordnung eine Matratzenschale (41) zum Halten der Matratze (42) aufweist, wobei die Mehrzahl der Heizzellen (61) in der Matratze angeordnet sind.

10. Vorrichtung nach Anspruch 9, wobei das mindestens eine Heizelement aus mindestens zwei getrennten Heizsegmenten gebildet ist.

11. Vorrichtung nach Anspruch 9, wobei das mindestens eine Heizelement aus mindestens drei getrennten Heizsegmenten gebildet ist.

## Revendications

1. Dispositif comprenant :
un ensemble de support de patient,
un matelas (42) disposé sur l'ensemble de support de patient, et
au moins un élément chauffant (60) constitué d'une pluralité de cellules chauffantes (61) disposées dans le matelas,
chaque cellule de la pluralité de cellules chauffantes (61) comprenant au moins un élément chauffant globalement plat (62) qui peut individuellement être alimenté ou non alimenté, et au moins un capteur de température (63),
**caractérisé en ce que** :
ledit au moins un élément chauffant (60) est divisé en une pluralité de cellules chauffantes (61) de manière à former une matrice globalement plate.

2. Dispositif selon la revendication 1, dans lequel la pluralité de cellules chauffantes sont disposées en étant adjacentes les unes aux autres dans une matrice.

3. Dispositif selon la revendication 1, comprenant en outre :
un bac à matelas destiné à recevoir le matelas, ledit bac à matelas étant couplé à l'ensemble de support de patient.

4. Dispositif selon la revendication 3, comprenant en outre une cassette à rayons X disposée en dessous du bac à matelas.

5. Dispositif selon la revendication 1, dans lequel chaque cellule de la pluralité de cellules chauffantes est formée à partir d'un matériau transparent aux rayons X.

6. Dispositif selon la revendication 1, dans lequel le au moins un élément chauffant est constitué d'une pluralité de segments chauffants.

7. Dispositif selon la revendication 6, dans lequel le au moins un élément chauffant est constitué d'au moins deux segments chauffants séparés.

8. Dispositif selon la revendication 6, dans lequel le au moins un élément chauffant est constitué d'au moins trois segments chauffants séparés.

9. Dispositif selon la revendication 1, comprenant en outre :
un ensemble de bac à matelas (40) couplé à l'ensemble de support de patient, ledit ensemble de bac à matelas incluant un bac à matelas (41) destiné à contenir un matelas (42), dans lequel la pluralité de cellules chauffantes (61) sont disposées dans le matelas.

10. Dispositif selon la revendication 9, dans lequel le au moins un élément chauffant est constitué d'au moins deux segments chauffants séparés.

11. Dispositif selon la revendication 9, dans lequel le au moins un élément chauffant est constitué d'au moins trois segments chauffants séparés.
